# EUROPEAN PATENT APPLICATION

(11) **EP 4 339 884 A1**
(43) Date of publication of application: **20.03.2024**
(21) Application number: 22306365.2
(22) Date of filing: 16.09.2022
(51) Int. Cl.: G06T 7/00, G06V 10/764, G06V 10/82, G06V 40/10

(54) **METHOD FOR ULTRASOUND IMAGE PROCESSING AND APPARATUS FOR IMPLEMENTING THE SAME**

(71) Applicant: Fondation de Coopération Scientifique, 67000 Strasbourg (FR)
(72) Inventor: FLEURENTIN, Antoine; Jean Michel, 49000 ANGERS (FR); MAZELLIER, Jean-Paul, 67000 STRASBOURG (FR); MEYER, Adrien, 67000 STRASBOURG (FR); PADOY, Nicolas, 67000 STRASBOURG (FR); SOSA VALENCIA, Leonardo, 67000 STRASBOURG (FR)
(74) Representative: Plasseraud IP

(57) **Abstract**

A method for processing a set of ultrasound images generated for observing a target organ is proposed, which comprises, for a result image of the set of ultrasound images: determining a prediction of a class of the result image using a first machine learning algorithm, wherein the class belongs to a set of classes comprising at least one class related to the target organ; determining whether the class is related to the target organ; and based on a determination that the class is related to the target organ, using a prediction of detection of presence, in the result image, of a structure in relation with a procedure for observing the target organ, determined based on the set of ultrasound images to generate an output signal.

## Description

The present disclosure relates to the field of image processing, in particular for ultrasound video stream processing.

Endoscopic ultrasound (EUS) and magnetic resonance imaging (MRI) are today considered as the most accurate tools for organ imaging (e.g. pancreatic imaging). In general, EUS is considered to be superior for detecting small lesions (≤ T1 tumors). Despite its strong potential, there is a general lack of access to high quality EUS which restricts its usefulness. Indeed, EUS is very difficult to master and requires demanding technical skills as well as a level of training and volume of experience significantly beyond that of general endoscopy. The difficulty of performing quality EUS procedures is related to two main issues: difficulty in understanding the orientation and location of the scope tip within the patient (technical skills), and the correct interpretation of the ultrasound images (interpretational skills).

Artificial intelligence (AI), and Deep Learning (DL) in particular, hold the promise of improving EUS practice by assisting the physician to identify structures in the ultrasound images. DL algorithms, especially convolutional neural networks (CNN), are a great asset for structure recognition as they can be trained to learn and then identify relevant features from images without explicit human intervention.

Nevertheless, schemes for providing improved EUS procedures through combining with AI-based technologies have yet to be proposed.

There is therefore a need for an improved ultrasound video processing scheme and apparatus implementing the same that address at least some of the above-described drawbacks and shortcomings of the conventional technology in the art.

It is an object of the present subject disclosure to provide an improved ultrasound video processing scheme and apparatus implementing the same.

Another object of the present subject disclosure is to provide an improved scheme for processing a set of ultrasound images scheme and apparatuses implementing the same.

Another object of the present subject disclosure is to provide an improved method for processing a set of ultrasound images generated, for example by ultrasound endoscopy, for observing a target organ and apparatuses implementing the same for alleviating the above-described drawbacks and shortcomings of conventional video encoding schemes.

To achieve these objects and other advantages and in accordance with the purpose of the present subject disclosure, as embodied and broadly described herein, in one aspect of the present subject disclosure, a method for processing a set of ultrasound images generated, for example by ultrasound endoscopy, for observing a target organ, is proposed. The proposed method comprises, for a result image of the set of ultrasound images: determining a prediction of a class of the result image using a first machine learning algorithm, wherein the class belongs to a set of classes comprising at least one class related to the target organ; determining whether the class is related to the target organ; based on a determination that the class is related to the target organ, using a prediction of detection of presence of a structure in relation with a procedure for observing the target organ in the result image determined based on the set of ultrasound images to generate an output signal, such as comprising, for example, one or more of an audio signal and an augmented result image.

In some embodiments, the proposed scheme provides the use of a machine learning algorithm for determining a prediction of a class of a result image in a set of ultrasound images, and determining whether the class is related to the target organ, prior to using another prediction to generate an output signal such as an augmented result image.

Therefore, advantageously, the determination of a prediction of the class of the result image may be used to first confirm that the result image is related to the target organ, before proceeding further and using a prediction of detection of presence of a structure in relation with a procedure for observing the target organ in the result image determined based on the set of ultrasound images to generate an output signal such as an augmented result image. In particular, the proposed scheme allows avoiding generating an output signal such as an augmented result image using the prediction of detection of presence of a structure in the result image in instances where the result image is actually not related to the target organ.

As another advantage, the proposed scheme may be used for processing ultrasound images generated by ultrasound endoscopy for observing a target organ, that is, in the context of EUS imaging. In such context, the proposed scheme may lead to significant improvements in the detection of pancreatic cancer, as one of the difficulties of using EUS imaging for observing a pancreas organ, besides the difficulties inherent to mastering EUS as mentioned above, relates to the need to detect small lesions and to ensure that detected lesions are indeed formed on the pancreas organ.

In one or more embodiments, the set of ultrasound images may be generated by ultrasound endoscopy. However, the present subject disclosure is not limited to ultrasound images generated by endoscopy, and may be applied to a set of ultrasound images generated by any suitable image generation technology.

In one or more embodiments, the detection of presence, in the result image, of the structure in relation with the procedure for observing the target organ, may comprise a detection of presence of tissues and/or a lesion of the target organ in the result image. For example, in some embodiments, a detection of presence of tissues may comprise an area (e.g. a box with a square or rectangular form) identified on the result image, a detection class (e.g. presence of pancreas) associated with the area, and possibly a detection score (e.g. a likelihood value) measuring a confidence level, and a detection of presence of lesion may comprise an area (e.g. a box with a square or rectangular form) identified on the result image, a detection class (e.g. presence of a lesion) associated with the area, and possibly a detection score (e.g. a likelihood value) measuring a confidence level.

In one or more embodiments, the prediction of detection may be determined by the first machine learning algorithm or by a second machine learning algorithm.

In one or more embodiments, the prediction of detection may be determined by a second machine learning algorithm, and the first machine learning algorithm and the second machine learning algorithm may be executed in parallel to generate, based on the set of ultrasound images, the prediction of the class and the prediction of the detection. Executing the first and second machine learning algorithm advantageously speeds up the completion of the first and second machine learning algorithm processing.

In one or more embodiments, the first machine learning algorithm may be configured to receive as input data preprocessed images of the set of ultrasound images.

In one or more embodiments, the first machine learning algorithm may be configured for implementing an artificial intelligence algorithm using a neural network.

In one or more embodiments, the prediction of detection may be determined by a second machine learning algorithm, and wherein the second machine learning algorithm may be configured to receive as input data preprocessed images of the set of ultrasound images.

In one or more embodiments, the output signal may comprise one or more of an audio signal, an augmented result image, and any other suitable multimedia signal.

In one or more embodiments, the first machine learning algorithm may be a supervised learning algorithm, and the proposed may further comprise: performing a training phase for training the supervised learning algorithm as a classification algorithm during which the supervised learning algorithm is trained with training data.

In one or more embodiments, the target organ is the pancreas.

In one or more embodiments, the augmented result image may be generated by superimposing on the result image information related to the presence of tissues of the target organ and/or the presence of a lesion of the target organ based on prediction of detection of the presence of tissues and/or a lesion of the target organ in the result image.

In one or more embodiments, the set of ultrasound images may be extracted from an ultrasound video stream generated by the ultrasound endoscopy for observing the target organ, and the proposed method may further comprise: displaying on a display an augmented ultrasound video stream comprising the augmented result image.

In one or more embodiments, the prediction of the class may comprise a likelihood value associated with the class, and the determining whether the class is related to the target organ may be based on a comparison of the likelihood value with a predefined threshold.

In another aspect of the present subject disclosure, an apparatus is proposed, which comprises a processor, and a memory operatively coupled to the processor, wherein the apparatus is configured to perform a method as proposed in the present subject disclosure.

In yet another aspect of the present subject disclosure, a non-transitory computer-readable medium encoded with executable instructions which, when executed, causes an apparatus comprising a processor operatively coupled with a memory, to perform a method as proposed in the present subject disclosure, is proposed.

For example, in some embodiments, the present subject disclosure provides a non-transitory computer-readable medium encoded with executable instructions which, when executed, causes an apparatus comprising a processor operatively coupled with a memory, to process a set of ultrasound images generated for observing a target organ, by performing a method for processing a set of ultrasound images, the method comprising, for a result image of the set of ultrasound images: determining, by the processor, a prediction of a class of the result image using a first machine learning algorithm, wherein the class belongs to a set of classes comprising at least one class related to the target organ; determining, by the processor, whether the class is related to the target organ; and based on a determination that the class is related to the target organ, using a prediction of detection of presence of a structure in relation with a procedure for observing the target organ in the result image determined based on the set of ultrasound images to generate an output signal such as, for example, an augmented result image.

In yet another aspect of the present subject disclosure, a computer program product comprising computer program code tangibly embodied in a computer readable medium, said computer program code comprising instructions to, when provided to a computer system and executed, cause said computer to perform a method as proposed in the present subject disclosure, is proposed.

In another aspect of the present subject disclosure, a data set representing, for example through compression or encoding, a computer program as proposed herein, is proposed.

It should be appreciated that the present invention can be implemented and utilized in numerous ways, including without limitation as a process, an apparatus, a system, a device, and as a method for applications now known and later developed. These and other unique features of the system disclosed herein will become more readily apparent from the following description and the accompanying drawings.

### Brief description of the drawings

The present subject disclosure will be better understood and its numerous objects and advantages will become more apparent to those skilled in the art by reference to the following drawings, in conjunction with the accompanying specification, in which:
Figure 1 illustrates an exemplary ultrasound image processing method according to one or more embodiments of the present subject disclosure;
Figure 2 is a block diagram illustrating an exemplary imaging system in accordance with one or more embodiments;
Figures 3a - 3b illustrate exemplary ultrasound image processing subsystems according to one or more embodiments of the present subject disclosure;
Figure 4 illustrates an exemplary apparatus or unit configured to use one or more features in accordance with one or more embodiments of the present subject disclosure.

### Description of embodiments

For simplicity and clarity of illustration, the drawing figures illustrate the general manner of construction, and descriptions and details of well-known features and techniques may be omitted to avoid unnecessarily obscuring the discussion of the described embodiments of the invention. Additionally, elements in the drawing figures are not necessarily drawn to scale. For example, the dimensions of some of the elements in the figures may be exaggerated relative to other elements to help improve understanding of embodiments of the present invention. Certain figures may be shown in an idealized fashion in order to aid understanding, such as when structures are shown having straight lines, sharp angles, and/or parallel planes or the like that under real-world conditions would likely be significantly less symmetric and orderly. The same reference numerals in different figures denote the same elements, while similar reference numerals may, but do not necessarily, denote similar elements.

In addition, it should be apparent that the teaching herein can be embodied in a wide variety of forms and that any specific structure and/or function disclosed herein is merely representative. In particular, one skilled in the art will appreciate that an aspect disclosed herein can be implemented independently of any other aspects and that several aspects can be combined in various ways.

The present disclosure is described below with reference to functions, engines, block diagrams and flowchart illustrations of the methods, systems, and computer program according to one or more exemplary embodiments. Each described function, engine, block of the block diagrams and flowchart illustrations can be implemented in hardware, software, firmware, middleware, microcode, or any suitable combination thereof. If implemented in software, the functions, engines, blocks of the block diagrams and/or flowchart illustrations can be implemented by computer program instructions or software code, which may be stored or transmitted over a computer-readable medium, or loaded onto a general purpose computer, special purpose computer or other programmable data processing apparatus to produce a machine, such that the computer program instructions or software code which execute on the computer or other programmable data processing apparatus, create the means for implementing the functions described herein.

Embodiments of computer-readable media includes, but are not limited to, both computer storage media and communication media including any medium that facilitates transfer of a computer program from one place to another. As used herein, a "computer storage media" may be any physical media that can be accessed by a computer or a processor. In addition, the terms "memory" and "computer storage media" include any type of data storage device, such as, without limitation, a hard drive, a flash drive or other flash memory devices (e.g. memory keys, memory sticks, key drive), CD-ROMs or other optical data storage devices, DVDs, magnetic disk data storage devices or other magnetic data storage devices, data memory components, RAM, ROM and EEPROM memories, memory cards (smart cards), solid state drive (SSD) memories, and any other form of medium able to be used to transport or store or memorize data or data structures able to be read by a computer processor, or a combination thereof. Furthermore, various forms of computer-readable media may transmit or carry instructions to a computer, such as a router, a gateway, a server, or any data transmission equipment, whether this involves wired transmission (via coaxial cable, optical fibre, telephone wires, DSL cable or Ethernet cable), wireless transmission (via infrared, radio, cellular, microwaves) or virtualized transmission equipment (virtual router, virtual gateway, virtual tunnel end, virtual firewall). According to the embodiments, the instructions may comprise code in any computer programming language or computer program element, such as, without limitation, the languages of assembler, C, C++, Visual Basic, HyperText Markup Language (HTML), Extensible Markup Language (XML), HyperText Transfer Protocol (HTTP), Hypertext Preprocessor (PHP), SQL, MySQL, Java, JavaScript, JavaScript Object Notation (JSON), Python, and bash scripting.

Unless specifically stated otherwise, it will be appreciated that throughout the following description discussions utilizing terms such as processing, computing, calculating, determining, or the like, refer to the action or processes of a computer or computing system, or similar electronic computing device, that manipulate or transform data represented as physical, such as electronic, quantities within the registers or memories of the computing system into other data similarly represented as physical quantities within the memories, registers or other such information storage, transmission or display devices of the computing system.

The terms "comprise," "include," "have," and any variations thereof, are intended to cover a non-exclusive inclusion, such that a process, method, article, or apparatus that comprises a list of elements is not necessarily limited to those elements, but may include other elements not expressly listed or inherent to such process, method, article, or apparatus.

Additionally, the word "exemplary" is used herein to mean "serving as an example, instance, or illustration". Any embodiment or design described herein as "exemplary" is not necessarily to be construed as preferred or advantageous over other embodiments or designs. The terms "in particular", "for example", "example", "typically" are used in the present description to denote examples or illustrations of non-limiting embodiments that do not necessarily correspond to preferred or advantageous embodiments with respect to other possible aspects or embodiments.

The terms "operationally coupled", "coupled", "mounted", "connected" and their various variants and forms used in the present description refer to couplings, connections and mountings that may be direct or indirect, and comprise in particular connections between electronic equipment or between portions of such equipment that allow operations and modes of operation as described in the present description. In addition, the terms "connected" and "coupled" are not limited to physical or mechanical connections or couplings. For example, an operational coupling may include one or more wired connection(s) and/or one or more wireless connection(s) between two or more items of equipment that allow simplex and/or duplex communication links between the equipment or portions of the equipment. According to another example, an operational coupling or a connection may include a wired-link and/or wireless coupling for allowing data communications between a server of the proposed system and another item of equipment of the system.

"Server" or "platform" in the present subject disclosure means any (virtualized or non-virtualized) point of service or computer device or system performing data processing operations, one or more databases, and/or data communication functions. For example, and without limitation, the term "server" or the term "platform" may refer to a physical processor operationally coupled to associated communication, database and data storage functions, or refer to a network, a group, a set or a complex of processors and associated data storage and networking equipment, and to an operating system and one or more database system(s) and application software supporting the services and functions provided by the server. A server or platform may be configured to operate in or as part of a cloud computing environment. A computer device or system may be configured so as to send and receive signals, via wireless and/or wired transmission networks(s), or be configured so as to process and/or store data or signals, and may therefore operate as a server. Equipment configured so as to operate as a server may thus include, by way of non-limiting example, dedicated servers mounted on a rack, cloud-based servers, desktop computers, laptop computers, service gateways (sometimes called "box" or "home gateway"), multimedia decoders (sometimes called "set-top boxes"), integrated equipment combining various functionalities, such as two or more of the abovementioned functionalities. The servers may vary greatly in terms of their configuration or their capabilities, but a server will generally include one or more central processing unit(s) and a memory. A server may also include one or more item(s) of mass memory equipment, one or more electric power supply/supplies, one or more wireless and/or wired network interface(s), one or more input/output interface(s), one or more operating system(s), such as Windows Server, Mac OS X, Unix, Linux, FreeBSD, or an equivalent.

The terms "network" and "communication network" as used in the present description refer to one or more data links that may couple or connect possibly virtualized equipment so as to allow electronic data to be transported between computer systems and/or modules and/or other devices or electronic equipment, such as between a server and a client device or other types of devices, including between wireless devices that are coupled or connected via a wireless network, for example. A network may also include a mass memory for storing data, such as a NAS (network attached storage), a SAN (storage area network) or any other form of computer-readable or machine-readable medium, for example. A network may comprise, in full or in part, the Internet, one or more local area networks (LAN), one or more wide area networks (WAN), wired connections, wireless connections, cellular connections or any combination of these various networks. Similarly, subnetworks may use various architectures or conform with or be compatible with various protocols, and interoperate with larger networks. Various types of equipment may be used to make various architectures or various protocols interoperable. For example, a router may be used to provide a communication link or a data link between two LANs that would otherwise be separate and independent.

Due in large part to a poor detection of early staged cancers, pancreatic cancer is currently in France the sixth leading cause of cancer-related mortality in France with a 15% 5 year survival rate and 11,456 deaths per year reported in 2018. Contrarily, early detection and treatment can result in survival rates of around 80%. Various strategies are being pursued for earlier pancreatic cancer detection: among them, advanced image-based diagnosis techniques such as Endoscopic Ultrasound (EUS) are particularly promising.

EUS is a minimally invasive procedure combining endoscopy with ultrasound to obtain detailed images of the internal organs of the chest, upper abdomen, or colon: it is considered today one of the best techniques for detecting pancreatic cancer lesions smaller than 20 mm, which area amenable for surgical resection and cure. Despite its strong potential, EUS is difficult to master and requires demanding technical skills as well as a level of training and volume experience significantly beyond that of basic endoscopy. This has led in turn to insufficient well-trained clinicians to provide this potentially life-saving technique to the population. The difficulty relates to two main points: spatial comprehension and diagnostic imaging.

In general, objects in natural images are easily identified by humans, based on their texture, color, shape and other elementary image features. Recognition of anatomical structures from EUS videos is more complicated, especially by observers with little experience. Structures such as blood vessels, the bile duct or a cyst, can be easily confused, even by a trained practitioner. Furthermore, boundaries of certain structures, in particular pancreatic parenchyma, are difficult to determine.

Despite all these challenges, Deep-Learning (DL) models still manage to detect lesions and pancreatic parenchyma ultrasound images generated by ultrasound endoscopy have been used for observing the pancreas of a patient. However, the performances of AI models (such as DL models) may not be as good for detecting objects of irregular structure, i.e., when the center of mass is difficult to determine, which is typically the case for pancreas parenchyma.

According to the present subject disclosure, a machine algorithm may advantageously be used to complement usage of a DL model (e.g. a CNN) trained for detecting presence of a structure in relation with a procedure for observing the target organ in a result image extracted from a set of ultrasound images with a classifier, that is, an AI model configured for classifying the result image in a class of images related to the target organ or in a class of images not related to the target organ.

In some embodiments, machine algorithm may be used for performing a preliminary post-processing of the result image prior to subjecting the image to processing by the DL model. The machine algorithm may be configured, e.g. by training, to determine a prediction of a class of the result image, which prediction may be used to determine whether or not the result image is related to the target organ.

In some embodiments, a preliminary sorting of result images being processed may be performed to distinguish between the result images which are related to the target organ, and the result images which are not.

In some embodiments, only the results image which have been determined as being related to the target organ may be subjected to further processing, for example comprising generating an augmented result image. Results image which have been determined as not being related to the target organ may for example be discarded.

The methods proposed in the present subject disclosure may be implemented by any computer system configured for image processing using an AI model, such as a convolutional neural network.

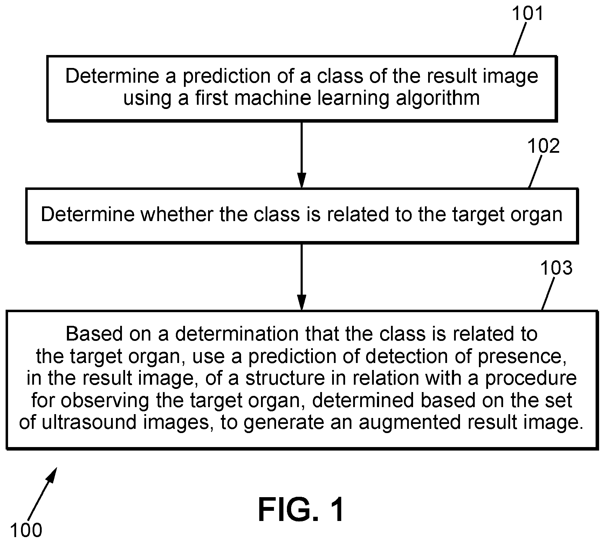

Figure 1 illustrates an exemplary image processing method 100 according to one or more embodiments of the present subject disclosure.

An ultrasound image which is to be processed is considered as input of the proposed method, and may be indifferently referred to in the following as the result image, the "original" image, or the "input" image. The result image may be selected in a set of ultrasound images which may indifferently be referred to in the following as the ultrasound video sequence, the "original" video sequence, or the "input" video sequence.

In one or more embodiments, the set of ultrasound images may have been generated as part of a procedure for observing a target organ, for example using ultrasound endoscopy. The result image may be selected based on any suitable selection criterion, for example chosen in view of the applicable imagery application. For example, video batches of a predetermined duration may be periodically extracted from an ultrasound video stream generated by ultrasound endoscopy, and the result image may be selected from an extracted batch.

In one or more embodiments, a prediction of a class of the result image may be determined (101) using a first machine learning algorithm. The class may belong to a set of classes which comprises at least one class related to the target organ.

In some embodiments, a set of classes may be defined, and a machine learning algorithm may be configured (e.g. through training) for predicting, based on input image data, one or more classes (belonging to the set of predefined classes) that correspond to the input image data.

At least one of the predefined classes may be defined as being related to the target organ. For example, the predefined set of classes may be defined to include a first subset of classes that are related to the target organ, and a second subset of classes that are not related to the target organ.

In one or more embodiments, data of the result image may be fed as input data, possibly after some pre-processing, for example for purposes of reformatting, to the first machine learning which may produce as output a class prediction.

In some embodiments, the result image may be divided into blocks or may be subjected to any suitable preprocessing for purposes of being fed to the machine learning algorithm in view of the configuration thereof.

In one or more embodiments, a determination (102) may be made as to whether the predicted class is related to the target organ.

For example, in some embodiments, it may be determined whether the predicted class belongs to a subset of classes that are related to the target organ, or else to a subset of classes that are not related to the target organ.

In one or more embodiments, the target organ is a human organ, for example the pancreas.

In one or more embodiments, in cases where the predicted class is determined to be related to the target organ, a prediction of detection of presence in the result image of a structure in relation with a procedure for observing the target organ may be obtained, and an output signal may be generated using the prediction of detection of presence in the result image. Depending on the embodiment, the output signal may comprise one or more of an audio signal, a video signal, and any other multimedia signal suitable for indicating the detection of presence in the result signal.

In one or more embodiments, in cases where the predicted class is determined to be related to the target organ, a prediction of detection of presence in the result image of a structure in relation with a procedure for observing the target organ may be obtained, and an augmented result image may be generated using the prediction of detection of presence in the result image (103).

In one or more embodiments, the set of ultrasound images may be extracted from an ultrasound video stream generated by ultrasound endoscopy for observing the target organ, and an augmented ultrasound video stream comprising the augmented result image may be displayed on a display device.

In some embodiments, a prediction of presence in the result image of tissues of the target organ and/or a lesion of the target organ may be obtained based on a determination that the class is related to the target organ.

In some embodiments, the detection of presence, in the result image, of the structure in relation with the procedure for observing the target organ, may comprise a detection of presence of tissues and/or a lesion of the target organ, or a detection of presence of landmarks located in the neighborhood of the target organ, in the result image. In such embodiments, the augmented result image may be generated by superimposing on the result image information related to the presence of tissues of the target organ and/or the presence of a lesion of the target organ based on prediction of detection of the presence of tissues and/or a lesion of the target organ in the result image.

In some embodiments, the detection may be configured to pertain to a target organ which may be any type of human organ, such as, for example, a pancreas, a blood vessel, a duct (e.g. bile duct type), a lymph node, etc. In other embodiments, the detection may be configured to pertain to the environment of the target organ, such as, for example, a landmark located in the neighborhood of the target organ. In yet other embodiments, the detection may be configured to pertain to structures other than the target organ itself, such as, for example, an object linked to a procedure performed by a practitioner, such as, for example, a biopsy needle.

In one or more embodiments, the prediction of detection of presence in the result image of a structure in relation with a procedure for observing the target organ may be generated by a machine learning algorithm executed based on input data comprising data of the result image, possibly after pre-processing.

Depending on the embodiment, the prediction of detection may be determined by the first machine learning algorithm or by a second machine learning algorithm. Therefore, in one or more embodiments, a first machine learning algorithm configured as a classifier, and a second machine learning algorithm configured as a detector, may be used for implementing the proposed method. In some embodiments, the first and second machine learning algorithms may be executed in parallel to generate, based on the set of ultrasound images, the prediction of the class and the prediction of the detection, so as to advantageously improve the response time of the system implementing the proposed process.

In some embodiments, the procedure for observing the target organ may be an endoscopy ultrasound imaging procedure, other ultrasound procedures or a magnetic resonance imaging procedure.

In some embodiments, the first machine learning algorithm is configured to receive as input data preprocessed images of the set of ultrasound images. The pre-processing performed on images of the set of ultrasound images may comprise resizing, converting to tensor, normalizing, and/or rescaling one or more images.

In embodiments where the prediction of detection is determined by a second machine learning algorithm, the second machine learning algorithm may also be configured to receive as input data preprocessed images of the set of ultrasound images. Such pre-processing performed on images of the set of ultrasound images may also comprise, depending on the embodiment, resizing, converting to tensor, normalizing, and/or rescaling one or more images.

In some embodiments, one or more of the following pre-processing operations may be used for data provided as input to the first machine learning algorithm and/or second machine learning algorithm: A resizing preprocessing operation may advantageously be used for reducing the size of the image(s) to be processed, for reducing the computation complexity of the processing. A conversion to tensor preprocessing operation may advantageously be used to optimize the format of the data to be processed when processing is implemented, at least in part, on a graphic card or using a graphic processor unit (GPU) or, depending on the embodiment, any processing unit, for example dedicated to neural network calculation (VPU, TPU, NPU, etc.). A normalizing of the color space preprocessing operation may advantageously be used to reduce the range of values used for color coding so as to improve the processing performances by reducing the computation complexity of the processing. In some embodiments, the preprocessing operations used during an inference phase of the second machine learning algorithm may advantageously also be used during a training phase of the second machine learning algorithm, as the case may be.

In one or more embodiments in which a detection of presence of tissues is used, one or more classes of the set of classes may be defined as related to the target organ. For example, the one or more classes may be defined as related to respective parts of the target organ and/or respective areas of (and/or around) the target organ, and/or parts thereof. For example, in some embodiments, the set of the one or more classes may be defined based on a set of subparts of the target organ which corresponds to a partition of the target organs into subparts, possibly with an overlap between two neighboring subparts.

For example, in some embodiments, one or more classes may be defined as respectively corresponding to parts and/or areas of the target organ such as the head of the target organ, the body of the target organ, and the tail of the target organ. For example, in embodiments in which a target organ such as the pancreas is observed using ultrasound endoscopy images, the following classes respectively related to parts and/or areas of the pancreas may be defined: HEAD, BODY, and TAIL, or, as another example {#1 = HEAD ; #2 = BODY ; #3= TAIL ; #4 = OTHER ; #5 : ARTIFACTS}.

In one or more embodiments, once a determination that the predicted class is related to the target organ is made, a prediction of detection of presence of tissues and/or a lesion of the target organ may be determined in order to proceed with a more detailed observation and/or exploration of the target organ.

For example, in some embodiments, the detection of presence of tissues may be configured for detecting so-called "landmarks" that correspond to parts and/or areas of the target organ, such as, for example areas and/or organs of (and/or around) the target organ and/or parts thereof corresponding to the determined predicted class.

For example, in embodiments in which a target organ such as the pancreas is observed using ultrasound endoscopy images, and in which the following classes respectively related to areas of the pancreas are defined: HEAD, BODY, and TAIL, the detection of presence of tissues may be configured for detecting the following "landmarks" that correspond to areas or organs of (or around) the pancreas or parts thereof, defined for each of the above classes:

| **Class** | **Corresponding landmarks** |
|---|---|
| **Head of pancreas:** | Superior mesentric vein and superior mesenteric artery |
| | Duodenum |
| | Vena cava |
| | Ventral/dorsal attachment |
| **Body of pancreas:** | Splenic vessels (artery and vein) |
| | Aorta and celiac artery |
| | Jejunum |
| | Stomach walls |
| **Tail of pancreas** | Splenic vessels(artery and vein) |
| | Stomach wall |
| | Spleen |
| | Kidney left |
| | Adrenal gland left |

Other additional landmarks may include, for example, a landmark for the Main Pancreatic duct, Common Bile duct, Gallbladder.

Such landmarks may be defined for the detection of presence in the result image of a structure in relation with a procedure for observing the target organ, and the prediction of detection of presence may provide one or more predictions of detections of one or more of such landmarks.

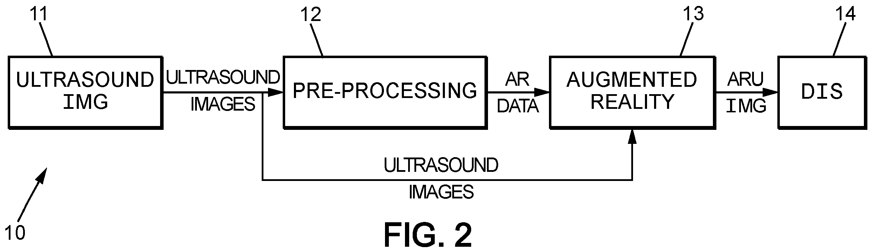

Fig. 2 shows an exemplary imaging system according to one or more embodiments of the subject disclosure.

Shown on Fig. 2 is a system 10 comprising an ultrasound image acquisition subsystem 11 configured to deliver ultrasound images a pre-processing subsystem 12 to which it is operatively coupled, for example via a data network (not represented on the Figure).

In one or more embodiments, the ultrasound image acquisition subsystem 11 may comprise an endoscopy ultrasound (EUS) system, which may be configured for generating an ultrasound video stream as part of an EUS procedure performed on a patient (e.g. a human patient) for observing an organ (e.g. the pancreas) of such patient.

Ultrasound images generated by the subsystem 11 are provided as input data to the pre-processing sub-system 12 and to an augmented reality sub-system 13, possibly through one or more data communication networks.

For example, in some embodiments, the ultrasound images may be input to the pre-processing sub-system 12 and/or the augmented reality sub-system 13 as uncompressed ultrasound video content which may be transported using a data communication link suitable for carrying raw video content, such as an SDI (Serial Digital Interface) link, a TSolP (Transport Stream over IP) link, a Digital Visual Interface (DVI) interface link or a HDMI (High-Definition Multimedia Interface) link, possibly using a multimedia interface splitter.

The pre-processing subsystem 12 may be configured to receive ultrasound image content (e.g. to receive an ultrasound video stream), and to process such content according to embodiments of the present subject-disclosure.

The pre-processing subsystem 12 may further be configured to generate as output data, based on the received ultrasound image content, augmented reality data for such content. As shown on Fig. 2, the augmented reality data generated by the pre-processing subsystem 12 may be provided to the augmented reality subsystem 13, through a suitable multimedia interface or a suitable data communication interface, possibly using a data communication network.

The augmented reality subsystem 13 may be configured for receiving the ultrasound image content generated by the ultrasound imaging subsystem 11 and the augmented reality data generated for such content by the pre-processing subsystem 12. The augmented reality subsystem 13 may be configured for generating, based on the received ultrasound image content and the received augmented reality data, augmented reality ultrasound images or, depending on the embodiment, and augmented reality ultrasound video stream, using conventional augmented reality techniques. The augmented reality ultrasound images or augmented reality ultrasound video stream may in some embodiments be fed to a display subsystem 14, so that, for example, they may be used to assist a practitioner performing a EUS procedure for observing an organ of a patient.

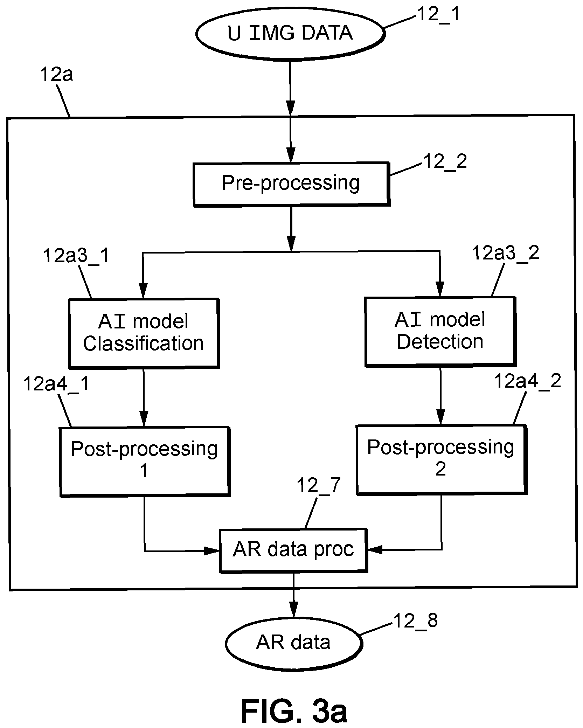

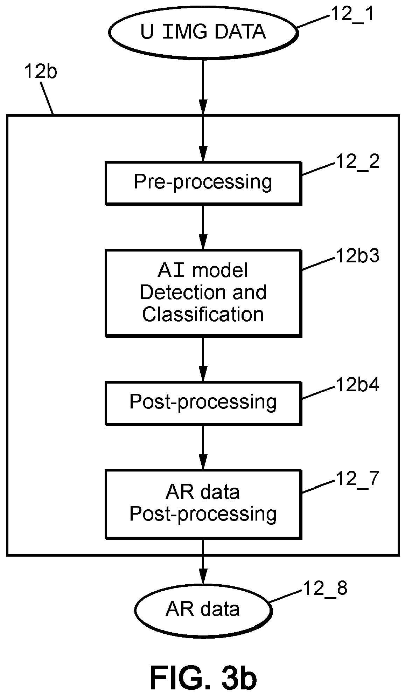

Further details of exemplary embodiments of the pre-processing subsystem 12 of Fig. 2 are provided in Fig. 3a and 3b.

Figures 3a and 3b show exemplary ultrasound image processing systems according to one or more embodiments of the subject disclosure. Depending on the embodiment, each of the exemplary ultrasound image processing system illustrated on Fig. 3a and Fig. 3b may be implemented in the pre-processing subsystem 12 of Fig. 2.

Figure 3a shows an exemplary ultrasound image processing system 12a according to some embodiments of the subject disclosure.

As shown on Fig. 3a, the ultrasound image processing system 12a may be configured to receive as input data, through an input interface (not shown on the figure) ultrasound image content or ultrasound image data 12_1, for example included in a received ultrasound video stream.

In some embodiments, the input data received by the ultrasound image processing system 12a may comprise a set of ultrasound images generated for observing a target organ, and the ultrasound image processing system 12a may be configured for processing the received set of ultrasound images according to embodiments of the present subject disclosure.

In some embodiments, one or more image processing operations may be performed by a pre-processing unit 12_2 on the image data received by the system 12a in order to generate input image data that can be input to a first artificial intelligence (AI) model 12a3_1 configured to implement a first machine learning algorithm, and to a second artificial intelligence model 12a3_2 configured to implement a second machine learning algorithm. Depending on the embodiment, the pre-processing unit 12_2 may be configured to perform image pre-processing operations which comprise an image resizing processing, a convert to tensor processing, a normalizing processing, and/or a rescaling processing.

In some embodiments, one or more of the following pre-processing operations may be used for data provided as input to the first machine learning algorithm (first artificial intelligence (AI) model 12a3_1) and/or second machine learning algorithm (second artificial intelligence model 12a3_2): A resizing preprocessing operation may advantageously be used for reducing the size of the image(s) to be processed, for reducing the computation complexity of the processing. A conversion to tensor preprocessing operation may advantageously be used to optimize the format of the data to be processed when processing is implemented, at least in part, on a graphic card or using a graphic processor unit (GPU) or, depending on the embodiment, any suitable processing unit, for example dedicated to neural network calculation (VPU, TPU, NPU, etc.). A normalizing of the color space preprocessing operation may advantageously be used to reduce the range of values used for color coding so as to improve the processing performances by reducing the computation complexity of the processing. In some embodiments, the preprocessing operations used during an inference phase of the second machine learning algorithm may advantageously also be used during a training phase of the second machine learning algorithm, as the case may be.

In some embodiments, different image processing operations may be performed on the image data received by the system 12a in order to generate input image data that can be input to the first artificial intelligence model 12a3_1 on the one hand, and to the second artificial intelligence model 12a3_2 on the other hand.

In one or more embodiments, the first machine learning algorithm may be configured as a classifier algorithm, to generate classification predictions based on ultrasound image data received as input data.

In some embodiments, the first machine learning algorithm may be configured to determine a prediction of a class of an ultrasound image received in input data among a predefined set of classes.

For example, in some embodiments, the first machine learning algorithm may be configured to determine that the ultrasound image received in input data belongs to one or more of the predefined set of classes.

In one or more embodiments, the predefined set of classes may comprise at least one class related to the target organ. For example, a class may be defined to include images on which the target organ (at least part thereof) can be identified. As another example, a class may be defined to include images on which a specific part of the target organ can be identified.

To the contrary, other classes may be defined to include images on which the target organ (at least part thereof) cannot be identified, or to include images on which a specific part of the target organ cannot be identified

Classes defined as corresponding to images in which presence of the target organ can be identified may be considered related to the target organ, whereas classes defined as corresponding to images in which presence of the target organ cannot be identified may not be considered related to the target organ.

In one or more embodiments, the first machine learning algorithm may be configured to generate, as a complement to the prediction of a class, a confidence level associated with the prediction (e.g. comprising a percentage indicating a likelihood that the prediction is correct).

In one or more embodiments, the second machine learning algorithm may be configured as a detection algorithm, to generate detection predictions based on ultrasound image data received as input data.

In some embodiments, the second machine learning algorithm may be configured to determine a prediction of a detection of presence, in the ultrasound image data 12_1, of a structure in relation with a procedure for observing the target organ.

For example, in some embodiments, the second machine learning algorithm may be configured to determine a prediction of a detection of presence, in the ultrasound image data 12_1, of tissues and/or a lesion of the target organ in the result image. For example, in some embodiments, the second machine learning algorithm may be configured to determine a prediction of a detection of presence which indicates that tissues of the target organ are present in the ultrasound image data 12_1, and possibly locations in the ultrasound image data 12_1 where the tissues of the target organ are detected as present and/or identifications of parts of the target organ detected as present in the ultrasound image data 12_1, and/or that a lesion of the target organ is present in the ultrasound image data 12_1, and possibly a location in the ultrasound image data 12_1 where the lesion of the target organ is detected as present and/or identification of a part of the target organ in which the lesion is detected as present in the ultrasound image data 12_1.

In one or more embodiments, the classification prediction and, depending on the embodiment, the associated confidence level, may be provided to an AR data post-processing unit 12_7 configured for processing the data output by the first AI model unit 12a3_1 and the second AI model unit 12a3_2.

In some embodiments, one or more post-processing operations are performed by a post-processing unit 12a4_1 on the classification prediction data generated by the first AI model unit 12a3_1 before such are provided to the AR data post-processing unit 12_7. Depending on the embodiment, the post-processing operations performed on classification predictions generated by the first AI model unit 12a3_1 may comprise processing for maximizing the prediction.

For example, in some embodiments, the classification prediction data may comprise one or more predicted classes, and a prediction score (e.g. a likelihood value) generated for each predicted class. For example, in embodiments in which the pancreas is the target organ being observed, the predicted classes may comprise the 5 above-discussed classes (defined as follows: { #1 = HEAD ; #2 = BODY; #3= TAIL; #4 = OTHER; #5: ARTIFACTS}). A prediction score, for example corresponding to a confidence level, may be obtained for each of the predicted classes (e.g. 60% confidence level for a pancreas head class (#1 = HEAD), and 40% confidence level for a pancreas body part (#2 = BODY)), and the prediction may be maximized by retaining for subsequent use the class with the highest prediction score.

In some embodiments, the data generated by the post-processing unit 12a4_1 may comprise label data corresponding to the class having the highest score with its score (e.g. "BODY [50%]").

In some embodiments, one or more post-processing operations are performed by a post-processing unit 12a4_2 on the detection prediction data generated by the second AI model unit 12a3_2 before such are provided to the AR data post-processing unit 12_7.

Depending on the embodiment, the post-processing operations performed on classification predictions generated by the second AI model unit 12a3_2 may comprise prediction filtering based on confidence threshold, and no maxima suppression processing.

In some embodiments, a prediction filtering of detection prediction data based on confidence threshold may comprise the following operations: any predicted class having a low confidence level (e.g. a confidence level below a predetermined threshold) may be discarded. A respective threshold may be chosen for each class or for each group of classes. For example, a threshold used for classes related to detection of pancreas may be higher than a threshold used for classes related to detection of a lesion, so as to advantageously increase the likelihood of detecting all pancreas lesions, potentially at the expenses of false positive detections.

In some embodiments, a no maxima suppression processing may comprise the following operations: predicted classes may be analyzed to detect redundancies, and such redundancies may be discarded. For example, if an object (e.g. a lesion) is detected several times, only a determined number of predicted classes with the highest prediction scores may be retained for subsequent use. In some embodiments, the predicted classes identified as redundant (for example corresponding to respective prediction boxes on the image with an intersection that is higher than a predetermined threshold) may be grouped together, and only a predetermined number of classes (e.g. one) having the highest prediction score(s) may be retained for subsequent use.

In some embodiments, the data generated by the post-processing unit 12a4_2 may comprise label data such as described above in connection with the post-processing unit 12a4_1.

Therefore, in one or more embodiments, an AI model unit 12a3_1 configured for classification and an AI model unit 12a3_2 configured for detection may be running in parallel based on ultrasound image data 12_1 received as input data by the system 12a, possibly after respective pre-processing operations before feeding the input data into their respective AI model unit, to produce classification prediction data (for the AI model unit 12a3_1) and detection prediction data (for the AI model unit 12a3_2) that are fed to the AR data processing unit 12_7, possibly after respective post-processing operations.

In one or more embodiments, the AR data post-processing unit 12_7 may be configured for, upon receiving the classification prediction data (possibly post-processed) comprising a predicted class and possibly a confidence level, determining whether the predicted class is related to the target organ.

In some embodiments where the first AI model unit 12a3_1 is configured for generating a confidence level associated with the predicted class, the determining whether the predicted class is related to the target organ may comprise comparing the confidence level with a predetermined threshold.

In some embodiments, the AR data post-processing unit 12_7 may be configured for, based on determining that the predicted class is related to the target organ, determining that the detection prediction data generated by the second AI model unit 12a3_2 may be used for purposes of generating augmented reality data 12_8 related to the ultrasound image data 12_1.

For example, in some embodiments, the first AI model unit 12a3_1 may be configured to predict, based on input data, a class associated with the ultrasound image data 12_1, between two predefined classes, one indicating that the target organ is present on the input image data, and the other one indicating that the target organ is not present on the input image data. In such embodiments, in a case where the class predicted by the first AI model unit 12a3_1 for the ultrasound image data 12_1 is the class indicating that the target organ is present on the input image data, the detection prediction generated by the second AI model unit 12a3_2 may be considered confirmed with respect to presence in the ultrasound image data 12_1 of the target organ, and such detection prediction may be used to generate the AR data 12_8 to be used for generating an augmented reality ultrasound image data. Otherwise, in a case where the class predicted by the first AI model unit 12a3_1 for the ultrasound image data 12_1 is the class indicating that the target organ is not present on the input image data, the detection prediction generated by the second AI model unit 12a3_2 may be considered invalid with respect to presence in the ultrasound image data 12_1 of the target organ, and such detection prediction may not be used to generate the AR data 12_8 to be used for generating an augmented reality ultrasound image data. Therefore, performances of the first AI model unit 12a3_1 configured to perform classification on input ultrasound image data may advantageously be leveraged for validating or invalidating the predictions generated by the second AI model unit 12a3_2 configured to perform detection on the input ultrasound image data.

Figure 3b shows another exemplary ultrasound image processing system 12b according to some embodiments of the subject disclosure.

As shown on Fig. 3b, the ultrasound image processing system 12b may also be configured to receive as input data, through an input interface (not shown on the figure) ultrasound image content or ultrasound image data 12_1, for example included in a received ultrasound video stream.

In some embodiments, the input data received by the ultrasound image processing system 12b may comprise a set of ultrasound images generated for observing a target organ, and the ultrasound image processing system 12b may be configured for processing the received set of ultrasound images according to embodiments of the present subject disclosure for generating as output data AR data 12_8 which may correspond in some embodiments with that described in connection with Fig. 3a.

For purposes of processing the received set of ultrasound images according to embodiments of the present subject disclosure, the ultrasound image processing system 12b shown on Fig. 3b includes a pre-processing unit 12_2, and an AR data post-processing unit 12_7 may correspond in some embodiments with that described in connection with Fig. 3a.

In contrast with the system of Fig. 3a, the ultrasound image processing system 12b shown on Fig. 3b includes a single AI model unit 12b3 which is configured for generating both detection prediction data and classification prediction data that may in some embodiments correspond to the detection prediction data generated by the AI model unit 12a3_2 of Fig. 3a and/or to the classification prediction data generated by the AI model unit 12a3_1 of Fig. 3a.

In some embodiments, prediction data generated by the AI model unit 12b3 may be processed by a post-processing unit 12b4, which may be configured for, depending on the embodiment, performing post-processing on prediction data such as processing for maximizing the classification prediction data , prediction filtering of detection prediction data based on confidence threshold, and/or no maxima suppression processing of detection prediction data.

For example, in some embodiments, a processing for maximizing the classification prediction data may comprise the following operations: classification prediction data may comprise one or more predicted classes, and a prediction score (e.g. a likelihood value) generated for each predicted class. The prediction score for each of the predicted classes (e.g. 60% confidence level for a pancreas head class, and 40% confidence level for a pancreas body part) may be obtained, and the classification prediction data may be maximized by retaining for subsequent use the class with the highest prediction score.

In some embodiments, a prediction filtering of detection prediction data based on confidence threshold may comprise the following operations: any predicted class having a low confidence level (e.g. a confidence level below a predetermined threshold) may be discarded. A respective threshold may be chosen for each class or for each group of classes. For example, a threshold used for classes related to detection of pancreas may be higher than a threshold used for classes related to detection of a lesion, so as to advantageously increase the likelihood of detecting all pancreas lesions, potentially at the expenses of false positive detections.

In some embodiments, a no maxima suppression processing may comprise the following operations: predicted classes may be analyzed to detect redundancies, and such redundancies may be discarded. For example, if an object (e.g. a lesion) is detected several times, only a determined number of predicted classes with the highest prediction scores may be retained for subsequent use. In some embodiments, the predicted classes identified as redundant (for example corresponding to respective prediction boxes on the image with an intersection that is higher than a predetermined threshold) may be grouped together, and only a predetermined number of classes (e.g. one) having the highest prediction score(s) may be retained for subsequent use.

The architecture of the system 12b advantageously avoids the use of two distinct AI model units, even running in parallel, and provides a gain in processing time which can be leveraged for achieving higher performances for an ultrasound image processing which operates in real-time or near real-time.

The proposed methods may be implemented by any image processing system or video processing system configured for processing images (or frames) of input image data or video data using an AI model, such as a deep learning model implemented by a machine learning algorithm, such as, for example an image processing system or video processing system configured for processing using a deep learning model that uses a neural network, such as, for example, a convolutional neural network, or an image processing system or video processing system configured for classification using a deep learning model that uses one or more autoencoders, as the case may be adapted for implementing one or more embodiments of the proposed methods.

In one or more embodiments, a training of the first ML algorithm used in the image processing system of the present subject disclosure may be performed during a training phase of the ML algorithm. The training phase may be designed for configuring the ML algorithm (e.g. determining weights of a neural network) as a classifier, given predefined classes, so that, during an inference phase, the first ML algorithm generates a prediction of class based on input image data.

In some embodiments, the first machine learning algorithm may be a supervised learning algorithm, and a training phase for training the supervised learning algorithm as a classification algorithm during which the supervised learning algorithm is trained with training data may be performed.

For example, the training phase may be designed according to any suitable scheme for training the first ML algorithm as a classifier, using any suitable training data, such as, for example a set of images annotated by medical personnel.

In some embodiments, several exemplary convolutional neural networks, such as Lenet5, vgg16, Mobilenet, Resnet or 13D, may be tested to compare their performances, for example in predicting a class of a given an input ultrasound image based on a set of predefined classes.

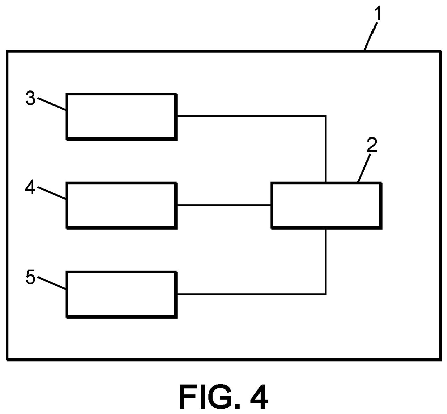

An exemplary architecture of an apparatus, such as a computer system, according to the present subject disclosure is illustrated on Fig. 4 which shows an apparatus 1 configured to perform a method for ultrasound image processing in accordance with embodiments of the present subject disclosure.

The apparatus 1, which may comprise one or more computers, includes a control engine 2, an ultrasound image processing engine 3, a data interface engine 4, a memory 5, and a prediction engine (not represented on Fig. 4).

In the architecture illustrated on Fig. 4, all of the ultrasound image processing engine 3, data interface engine 4, prediction engine, and memory 5 are operatively coupled with one another through the control engine 2.

In some embodiments, the ultrasound image processing engine 3 is configured to perform various aspects of embodiments of one or more of the proposed methods for ultrasound image processing as described herein, such as a prediction of a class of the result image using a first machine learning algorithm, and determining, based on the determination that the class is related to the target organ, that a prediction of detection of presence, in an input ultrasound image, of a structure in relation with a procedure for observing is target organ, determined based on a set of ultrasound images, is to be used for generating an augmented image of the input ultrasound image.

In some embodiments, the data interface engine 4 is configured to receive an input set of ultrasound images, possibly as part of an input ultrasound video stream, and to output augmented reality data for the generation of augmented reality ultrasound images based on input ultrasound images.

In some embodiments, the prediction engine may be configured for implementing a first artificial intelligence algorithm using a neural network, such as for example a supervised learning algorithm. The prediction engine may additionally be configured for implementing the functions or embodiments provided in the present subject disclosure with respect to training the first artificial intelligence algorithm or using the first artificial intelligence algorithm for obtaining classification predictions on input ultrasound image data.

The control engine 2 includes a processor, which may be any suitable microprocessor, microcontroller, Field Programmable Gate Arrays (FPGA), Application Specific Integrated Circuits (ASIC), Digital Signal Processing chip, and/or state machine, or a combination thereof. According to various embodiments, one or more of the computers can be configured as a multi-processor computer having multiple processors for providing parallel computing. The control engine 2 may also comprise, or may be in communication with, computer storage media, such as, without limitation, the memory 5, capable of storing computer program instructions or software code that, when executed by the processor, causes the processor to perform the elements described herein. In addition, the memory 5 may be any type of data storage or computer storage medium, coupled to the control engine 2 and operable with the data interface engine 4 and the ultrasound image processing engine 3 to facilitate management of data stored in association therewith, such as, for example, a cache memory, a data farm, a data warehouse, a data mart, a datacenter, a data cloud, or a combination thereof.

In embodiments of the present subject disclosure, the apparatus 1 is configured for performing one or more of the methods described herein. The apparatus 1 may in some embodiments be included in a video processor or, depending on the embodiments, in a FPGA component.

It will be appreciated that the apparatus 1 shown and described with reference to Fig. 4 is provided by way of example only. Numerous other architectures, operating environments, and configurations are possible. Other embodiments of the node may include fewer or greater number of components, and may incorporate some or all of the functionality described with respect to the apparatus components shown in Fig. 4. Accordingly, although the control engine 2, ultrasound image processing engine 3, data interface engine 4, prediction engine and memory 5 are illustrated as part of the apparatus 1, no restrictions are placed on the location and control of these components. In particular, in other embodiments, any of these components may be part of different entities or computing systems.

Embodiments of the proposed method for encoding image data provide several advantages, including the following in the context of EUS for pancreatic image processing:
By proposing solutions to overcome the two main limitations of EUS, the number of early stage pancreatic cancers identified can advantageously be increased, consequently improving the overall survival rate of patients.

To address issues around accurate image-based cancer diagnosis (cancer vs not-cancer, metastatic node vs normal mode, etc...), the present subject disclosure advantageously provides embodiments of a system configured for computer-assisted image recognition based on Artificial Intelligence (AI) for EUS operators. Depending on the embodiment, the proposed AI-based solution can advantageously be operated to facilitate lymph node detection, automatically assess their malignancy, and provide insights about predictive factors of pancreatic cancer, also assessing the quality of pancreatic parenchyma.

Depending on the embodiment, the detection of presence, which may be performed using an AI model (e.g. a neural network) configured for generating detection prediction data based on input image data, may be configured for operating the detection of lesions (e.g. solid, cystic, lymph nodes) and/or the detection of the type of blood vessels.

Depending on the embodiment, the classification performed using an AI model (e.g. a neural network) configured for generating classification prediction data based on input image data, may advantageously provide EUS operations improvements, such as assisted pancreas screening methods for legal protection with a quality report, and/or improving EUS skills for trainees and increasing self-confidence of EUS experts.

In addition, the use of an AI model configured as a classifier does not have, given the current state of the art of computer systems implementing AI technology, any significant impact on system performances, in particular in terms of response time, or computational complexity.

While the invention has been described with respect to preferred embodiments, those skilled in the art will readily appreciate that various changes and/or modifications can be made to the invention without departing from the spirit or scope of the invention as defined by the appended claims.

Although this invention has been disclosed in the context of certain preferred embodiments, it should be understood that certain advantages, features and aspects of the systems, devices, and methods may be realized in a variety of other embodiments. Additionally, it is contemplated that various aspects and features described herein can be practiced separately, combined together, or substituted for one another, and that a variety of combination and sub-combinations of the features and aspects can be made and still fall within the scope of the invention. Furthermore, the systems and devices described above may not include all of the modules and functions described in the preferred embodiments.

Information and signals described herein can be represented using any of a variety of different technologies and techniques. For example, data, instructions, commands, information, signals, bits, symbols, and chips can be represented by voltages, currents, electromagnetic waves, magnetic fields or particles, optical fields or particles, or any combination thereof.

Depending on the embodiment, certain acts, events, or functions of any of the methods described herein can be performed in a different sequence, may be added, merged, or left out all together (e.g., not all described acts or events are necessary for the practice of the method). Moreover, in certain embodiments, acts or events may be performed concurrently rather than sequentially.

## Claims

1. A method for processing a set of ultrasound images generated for observing a target organ, comprising, for a result image of the set of ultrasound images:
determining a prediction of a class of the result image using a first machine learning algorithm, wherein the class belongs to a set of classes comprising at least one class related to the target organ;
determining whether the class is related to the target organ; and
based on a determination that the class is related to the target organ, using a prediction of detection of presence, in the result image, of a structure in relation with a procedure for observing the target organ, determined based on the set of ultrasound images to generate an output signal.

2. The method according to claim 1, wherein the set of ultrasound images is generated by ultrasound endoscopy.

3. The method according to any of the preceding claims, wherein the detection of presence, in the result image, of the structure in relation with the procedure for observing the target organ, comprises a detection of presence of tissues and/or a lesion of the target organ in the result image.

4. The method according to any of the preceding claims, wherein the prediction of detection is determined by the first machine learning algorithm or by a second machine learning algorithm.

5. The method according to any of the preceding claims, wherein the prediction of detection is determined by a second machine learning algorithm, and wherein the first machine learning algorithm and the second machine learning algorithm are executed in parallel to generate, based on the set of ultrasound images, the prediction of the class and the prediction of the detection.

6. The method according to any of the preceding claims, wherein the first machine learning algorithm is configured to receive as input data preprocessed images of the set of ultrasound images.

7. The method according to any of the preceding claims, wherein the first machine learning algorithm is configured for implementing an artificial intelligence algorithm using a neural network.

8. The method according to any of the preceding claims, wherein the prediction of detection is determined by a second machine learning algorithm, and wherein the second machine learning algorithm is configured to receive as input data preprocessed images of the set of ultrasound images.

9. The method according to any of the preceding claims, wherein the output signal comprises an augmented result image.

10. The method according to any of the preceding claims, wherein the first machine learning algorithm is a supervised learning algorithm, the method further comprising: performing a training phase for training the supervised learning algorithm as a classification algorithm during which the supervised learning algorithm is trained with training data.

11. The method according to claim 9, wherein the augmented result image is generated by superimposing on the result image information related to the presence of tissues of the target organ and/or the presence of a lesion of the target organ based on prediction of detection of the presence of tissues and/or a lesion of the target organ in the result image.

12. The method according to any of claims 9 and 11, wherein the set of ultrasound images is extracted from an ultrasound video stream generated by the ultrasound endoscopy for observing the target organ, the method further comprising: displaying on a display an augmented ultrasound video stream comprising the augmented result image.

13. The method according to any of the preceding claims, wherein the prediction of the class comprises a likelihood value associated with the class, wherein the determining whether the class is related to the target organ is based on a comparison of the likelihood value with a predefined threshold.

14. An apparatus, the apparatus comprising a processor and a memory operatively coupled to the processor, wherein the apparatus is configured to perform a method according to any of claims 1 to 13.

15. A computer program product comprising computer program code tangibly embodied in a computer readable medium, said computer program code comprising instructions to, when provided to a computer system and executed, cause said computer to perform a method according to any of claims 1 to 13.
